# EUROPEAN PATENT APPLICATION

(11) **EP 4 549 552 A1**
(43) Date of publication of application: **07.05.2025**
(21) Application number: 23207224.9
(22) Date of filing: 31.10.2023
(51) Int. Cl.: C12N 5/077

(54) **CARDIAC FIBROBLAST CELL LINE CONTAINING KNOCKOUT OF THE FHL1 GENE**

(71) Applicant: Vilnius University, 01513 Vilnius (LT)
(72) Inventor: Baltriukiene, Daiva, Vilnius (LT); Baltrukonyte, Emilija, Vilnius (LT); Iesmantaite, Monika, Vilnius (LT); Bukelskiene, Virginija, Vilnius (LT)
(74) Representative: AAA Law

(57) **Abstract**

The invention relates to the method of generating immortalized cardiac fibroblasts with knockout *Fhl1* gene. The cells are useful in therapy as a model for finding new strategies to treat cardiomyopathy.

## Description

### FIELD OF THE INVENTION

The present invention relates to a method for generation of continuous Fhl1 knockout cell line of mice cardiac fibroblasts.

### BACKGROUND OF THE INVENTION

Fibrosis isa dysregulated tissue repair response when excessive accumulation of extracellular matrix (ECM) is observed. This can lead to alteration of tissue architecture, organ dysfunction or death (Henderson N. et al., 2020). It can affect the functioning of many organs, such as the lungs, kidney, heart, liver, etc. (Frangogiannis N., 2021). In the heart, fibrosis can be observed in most diseases, such as myocardial infarction, hypertensive heart disease and various types of cardiomyopathies. Excessive deposition of ECM increases the stiffness of the heart muscle and worsens cardiac functions (Jiang W. et al., 2021). The main cells involved in cardiac fibrosis are cardiac fibroblasts (CF). During fibrosis and the tissue repair process, fibroblasts are activated into myofibroblasts. Myofibroblasts are the main cellular effectors producing ECM (Jiang W. et al., 2021). Fibrosis is pathophysiologically heterogeneous and involves a complex process of fibroblast response involving different mechanisms, which makes it difficult to develop antifibrotic strategies or understand the mechanisms (Frangogiannis N., 2021). For this reason, there is still a need to identify new molecular targets involved in the mechanisms of cardiac fibrosis. One potential target of cardiac fibrosis is FHL1. FHL1 is a Four-and-a-half LIM domain protein 1, which belongs to the FHL protein family. This protein family is mainly found in muscle (Domenighetti A. et al., 2014). It is known that alterations in the Fhl1 gene play an important role in various myopathies (Wei X. and Zhang H., 2020). However, there is insufficient information on the role of FHL1 in cardiac fibrosis and whether this protein can be an antifibrotic target. There is also a lack of models for studying FHL1 in developing disease. Cardiac fibroblasts are primary cells used to study the fibrosis process. However, it is difficult to maintain cardiac fibroblasts in cell culture for long periods of time because their phenotype is very plastic, and they are sensitive to mechanical influences. One of the available approaches is to precisely control cell culture conditions (e.g. serum, nutrient concentration, and substrate), which are critical for controlling fibroblast proliferation over time (Landry et al., 2019).

US20180318405A1 describes compositions and methods for identifying and using agents capable of inhibiting myofibroblast transition and methods for treating fibrotic diseases.

Thus, a need for controlled fibroblast cell cultures that could be used as cell models for the analysis of cardiomyopathy and the development of new treatment strategies remains.

### SUMMARY OF THE INVENTION

The present invention focuses on the development of a reliable method for targeting the Fhl1 protein in cardiac tissue. Advantageously, the present invention provides a method that generates long-term cardiac fibroblasts.

The present invention provides a novel method for obtaining an immortalized cardiac fibroblast *Fhl1* knockout cell line that can serve as a continuous cell model for the analysis of cardiomyopathy and the development of new treatment strategies. The method comprises the following steps: a) obtaining primary cardiac fibroblasts; b) immortalization of fibroblasts; c) knockout of the *Fhl1* gene. The presently disclosed method provides an advantageous immortalized cardiac fibroblast Fhl1 knockout cell, that can be used in cell models for the analysis of cardiomyopathy and the development of new treatment strategies.

### BRIEF DESCRIPTION OF THE DRAWINGS

The drawings are provided as a reference to possible embodiments and are not intended to limit the scope of the invention. Neither of the drawings nor the graphs presented herein should be construed as limiting the scope of the invention, but merely as an example of a possible embodiment.
Fig. 1. Mouse cardiac fibroblasts 3 days after isolation (A) and passage No 3 (B). On the third day after cell isolation, the cells were elongated and spindle-shaped. In the third passage, the morphology of the cells changes, they become larger and more branched.
Fig. 2. Mouse cardiac fibroblasts grown 3 days after cell isolation on the surface of a conventional culture dish surface (A) and 3 days after cell isolation on surface coated with PDMS and vitronectin (B). There were more cells on the PDMS-coated surface. The cells were less branched and maintained the elongated shape typical of fibroblasts better than the cells that had grown on the conventional surface of the cell culture dish.
Fig. 3. Immortalized mouse cardiac fibroblasts after passage No. 21 retained high proliferation rate, were able to reach confluency and didn't change their morphology through passages.

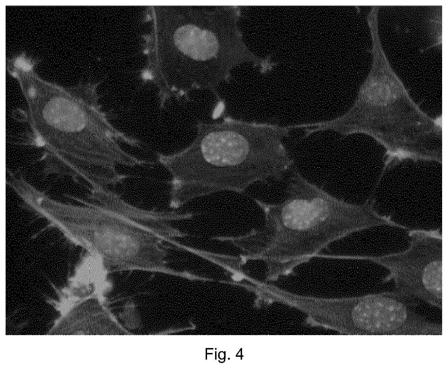

Fig. 4. Immunocytochemical analysis of vimentin-positive mouse cardiac fibroblasts grown on the PDMS-vitronectin coated surface. Vimentin expression can be seen in the cytoplasm of the cells, while the nuclei are stained white as round circles in the center of the cells. This confirms the quiescent state of the primary cardiac fibroblasts.
Fig. 5. Evaluation of senescence using β-galactosidase assay (shown as dark grey cells). A-B - SWISS 3T3, control; C-D - primary cardiac fibroblasts, 24 days after isolation; E-F - immortalized cardiac fibroblasts, passage No. 16. β-galactosidase activity was detected in primary cardiac fibroblasts (C-D), but there was no activity in immortalized cardiac fibroblasts (E-F).
Fig. 6. Evaluation of cell migration showed the capability of immortalized fibroblasts to migrate close the wound during 24 hours. Wound closure is expressed as a percentage of the closed area compared to the original area of the wound.

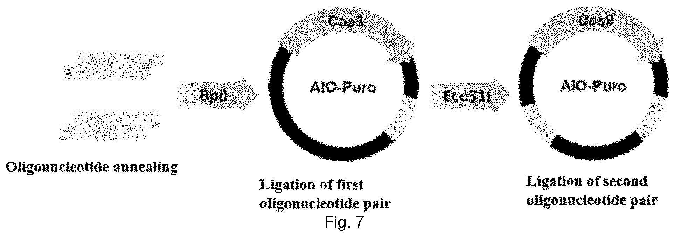

Fig. 7. Scheme of the plasmid for *Fhl1* knockout.
Fig. 8. Comparison of *Fhl1* gene expression analysis. mCF refers to cardiac mouse fibroblasts, mCF-SV40 - immortalized cardiac mouse fibroblasts, MT - mouse muscle tissue, HT - mouse heart tissue, Swiss 3T3 - embryonic mouse fibroblast line. Primers for both Fhl1 and Fhl2 were used.

### DETAILED DESCRIPTION OF THE INVENTION

The present invention focuses on the development of an advantageous reliable method for targeting the Fhl1 protein in cardiac tissue.

The present disclosure provides a method for generation of immortalized cardiac fibroblast *Fhl1* knockout cells, comprising the steps: a) obtaining primary cardiac fibroblasts, b) immortalizing the isolated fibroblasts, and c) producing *Fhl1* gene knockout in the cells.

In some embodiments, the cardiac fibroblasts that are generated by the presently disclosed method are mammal cardiac fibroblasts. In some embodiments, the cardiac fibroblasts are rodent cardiac fibroblasts. In preferred embodiments, the cardiac fibroblasts are mouse cardiac fibroblasts.

In some aspects, in the presently disclosed method for generation of immortalized cardiac fibroblast *Fhl1* knockout cells, after the step a) the cells are cultured on a surface coated with PDMS and vitronectin. Polydimethylsiloxane (PDMS), an elastomer with excellent optical, electrical and mechanical properties, is preferably coated with vitronectin.

In some aspects, in the presently disclosed method for generation of immortalized cardiac fibroblast *Fhl1* knockout cells the isolated fibroblasts are immortalized through viral-mediated induction of the large T-antigen. In some embodiments, the viral-mediated induction of the large T-antigen is made by using SV40 large T antigen.

In some aspects, in the presently disclosed method for generation of immortalized cardiac fibroblast *Fhl1* knockout cells the *Fhl1* gene knockout is produced using CRISPR-Cas system. In some embodiments, the CRISPR-Cas system is CRISPR-Cas9 nickase system.

Advantageously, the method of the present invention provides a strategy to attenuate senescence signals concurrently with SV40 expression to prevent spontaneous changes with subsequent downregulation of *Fhl1* by CRISPR-Cas9 nickase-mediated inactivation.

The present disclosure further provides an immortalized cardiac fibroblast *Fhl1* knockout cell. Preferably, the immortalized cardiac fibroblast *Fhl1* knockout cell is obtained by the method according to the present disclosure. In some embodiments, the immortalized cardiac fibroblast *Fhl1* knockout cell is obtained from mouse cardiac fibroblast.

The method of the present invention advantageously combines the steps of a) obtaining primary cardiac fibroblasts, b) immortalizing the isolated fibroblasts, and c) producing *Fhl1* gene knockout in the cells.

Various methods may be suitable for obtaining primary cardiac fibroblasts for use in the method for generation of immortalized cardiac fibroblast *Fhl1* knockout cells of the present disclosure. Immortalization of cardiac fibroblasts - the immortalized cells are capable of prolonged proliferation, but also have a similar or identical genotype and phenotype to their parental tissue, making them consistent material for a research project. There are several methods for immortalizing mammalian cells under culture conditions that are suitable for use in the step b) of the method of the present invention; these are, for example, the use of viral genes such as Simian virus 40 (SV40) T antigen, expression of telomerase reverse transcriptase protein (TERT), especially for cells most affected by telomere length such as human cells, co-expression of the hTERT catalytic subunit with either p53 or RB siRNA, etc. The immortalization procedure can be advantageously performed on cells as early as at four to six passages. Immortalization does not alter the morphology of the cardiac fibroblasts.

The term "knockout" is used herein to indicate disrupting a function of a target gene which exists in the genome. A gene that is knocked out stops its expression permanently and renders it non-functional. Methods used for producing knockout cells include Cre-LoxP, conventional homologous recombination or a CRISPR-Cas system.

### Examples

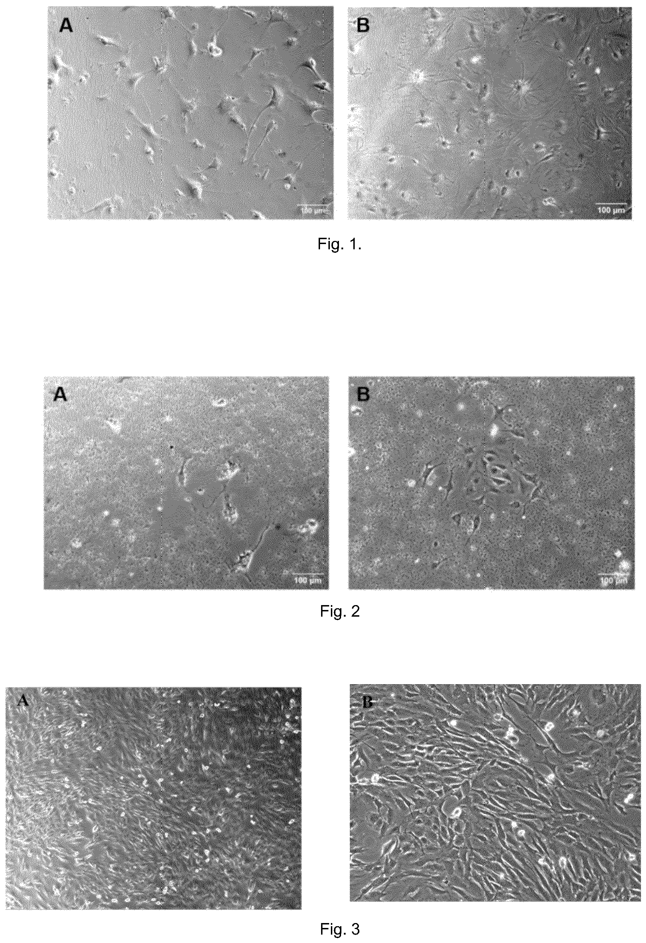

Isolation of cardiac mouse fibroblasts (Landry et al., 2019). Mouse cardiac fibroblasts were isolated from 4-6-week-old C57BL/6 mice. Shortly after sacrificing mice, when loss of limb reflexes is observed, the thorax was opened to remove the heart with part of the ascending aorta remaining attached. The heart was washed with 10 ml EDTA buffer (5 mM EDTA, 130 mM NaCl, 5 mM KCI, 500 nM NaH2PO4, 10 mM HEPES, 10 mM glucose, pH 7.8) and 3 ml perfusion buffer (1 mM MgCl2, 130 mM NaCl, 5 mM KCI, 500 nM NaH₂PO₄, 10 mM HEPES, 10 mM glucose, pH 7.8). A 29G needle inserted via the apex was used for the washes. After the washing procedures, the heart was injected through the apex with 10 ml collagenase type II (500x) diluted in HBSS with calcium and magnesium. The heart was then placed in fresh 10 ml collagenase type II solution and incubated for 1 hour at 37 °C and 300 rpm with shaking. After 1 hour, the heart was carefully pulled apart with forceps and digested for a further 10 minutes under the same conditions. Then the resulting suspension was neutralized with 10 ml of complete culture medium (DMEM/F12 (Dulbecco's Modified Eagle's medium/Ham's F12 nutrient mixture), 10 % FBS (Fetal Bovine Serum), 1 % penicillin/streptomycin), filtered through a 40 µm cell strainer and centrifuged at 200 × g for 7 minutes. The cell pellet was resuspended in 12 mL of complete culture medium, evenly distributed over 4 wells of a 6-well plate and the fibroblasts allowed to adhere for 2.5 hours at 37 °C and 5 % CO₂. After 3 hours, the cells were carefully washed with pre-warmed PBS supplemented with 3 % penicillin/streptomycin and the complete culture medium was added. The cells were washed with PBS supplemented with 3 % penicillin/streptomycin and the culture medium was then replaced once daily until the cells adopted a spindle-shaped morphology (~3-4 days, Fig. 1).

The isolated cells were cultured on a surface coated with PDMS (Merck) (Chuah et al., 2020) and 1.5 µg/mL vitronectin solution (Merck), for a final coating concentration of 0.5 µg/cm² and stable cell morphology was observed during the passages (Fig. 2).

The cells were maintained DMEM/F12 media supplemented with 10 % FBS, 1 % penicillin/streptomycin.

### Immortalization of cardiac fibroblasts

pHAGE_SV40 plasmids were used for immortalization of the cells by lentiviral transduction. After puromycin selection some of mouse cardiac fibroblasts cells survived and proliferated. Morphological evaluation was done using light microscopy. Cells were proliferating fast, and did not change their morphology through passages.

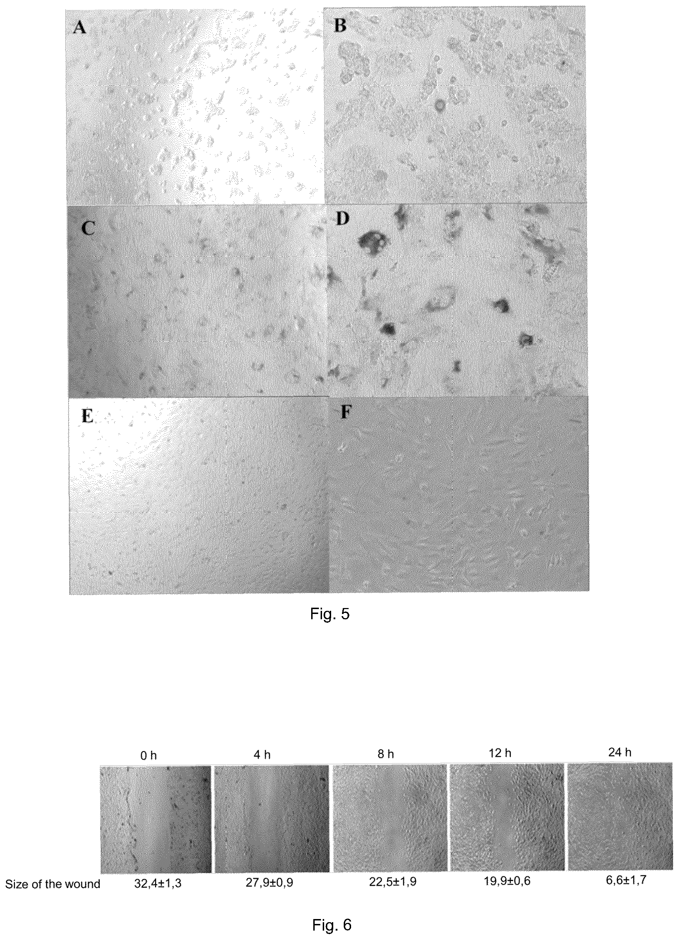

β-galactosidase assay (Merck) was used to evaluate cell senescence. Lysosomal β-galactosidase cleaves β-D-galactose residues into β-D-galactosides. Detectable β-galactosidase activity is the most commonly used marker of ageing or senescent cells, whether in culture or in mammalian tissue. Specifically, at a pH of 6.0, the enzyme β-galactosidase hydrolyses 5-bromo-4-chloro-3-indoyl-β-d-galactopyranoside, a colourless, soluble compound consisting of galactose bound to an indole. This reaction releases a deep blue, insoluble product on the cell culture or in the tissue (de Mera-Rodríguez et al., 2021) (in the Figure 5, the reaction product can be seen as a dark grey dots in the senescent cells).

Immortalized cell migratory potential was evaluated by the scratch assay (Liang et al., 2007).

Real-time quantitative polymerase chain reaction (RT-qPCR) was used to test *Fhl1* gene expression. The results showed that immortalization had no effect on the expression of the *Fhl1* gene (Fig. 8).

### Knockout of Fhl1 gene in cardiac fibroblasts

For *Fhl1* gene knockout Cas9 nickase system was chosen. Two-step construction of AIO-Puro plasmid (Addgene, Plasmid #74630) encoding Cas9 (D10A) nickase was performed. Two pairs are required for the CRISPR-Cas9 nickase, which causes a single-stranded DNA break at two sites. In the first step, the plasmid is restricted with Bpil restriction endonuclease, and the first pair of oligonucleotides is ligated. The second stage is performed analogously, only it is restricted with Eco31I restriction endonuclease, and the second pair of oligonucleotides is ligated.

Table of gRNA sequences used in knockout experiments:

| **Name** | **Forward 5' - 3' (FW)** | **Reverse 5' - 3' (RV)** |
|---|---|---|
| g1RNR-1 | ACCGGCTTGGGTACCTGCGGCCTC | AAACGAGGCCGCAGGTACCCAAGC |
| g1RNR-2 | ACCGGCACGAGCTGCGGCTCCCCG | AAACCGGGGAGCCGCAGCTCGTGC |
| g2RNR-1 | ACCGCTGCTGGGCTTGGGTACCTG | AAACCAGGTACCCAAGCCCAGCAG |
| g2RNR-2 | ACCGCGGCTCCCCGGGGACGCCGG | AAACCCGGCGTCCCCGGGGAGCCG |
| g3RNR-1 | ACCGCTGCTGGGCTTGGGTACCTG | AAACCAGGTACCCAAGCCCAGCAG |
| g3RNR-2 | ACCGGCACGAGCTGCGGCTCCCCG | AAACCGGGAGCCGCAGCTCGTGC |

### References

Chuah, Y.J., Heng, Z. T., Tan, J. S., Tay, L. M., Lim, Ch. S., Kang, Y., Wang, D.-A. Surface modifications to polydimethylsiloxane substrate for stabilizing prolonged bone marrow stromal cell culture. Colloids and Surfaces B: Biointerfaces 2020, 191:110995. doi.org/10.1016/j.colsurfb.2020.110995
de Mera-Rodríguez, J. A., Álvarez-Hernán, G., Gañán, Y., Martín-Partido, G., Rodríguez-León, J., Francisco-Morcillo, J. Is Senescence-Associated β-Galactosidase a Reliable in vivo Marker of Cellular Senescence During Embryonic Development? Front. Cell Dev. Biol., 2021, Sec. Cell Death and Survival, 9 -2021. doi.org/10.3389/fcell.2021.623175
Domenighetti AA, Chu PH, Wu T, Sheikh F, Gokhin DS, Guo LT, Cui Z, Peter AK, Christodoulou DC, Parfenov MG, Gorham JM, Li DY, Banerjee I, Lai X, Witzmann FA, Seidman CE, Seidman JG, Gomes AV, Shelton GD, Lieber RL, Chen J. Loss of FHL1 induces an age-dependent skeletal muscle myopathy associated with myofibrillar and intermyofibrillar disorganization in mice. Hum Mol Genet. 2014 Jan 1;23(1):209-25. doi: 10.1093/hmg/ddt412.
Frangogiannis NG. Cardiac fibrosis. Cardiovasc Res. 2021 May 25;117(6):1450-1488. doi: 10.1093/cvr/cvaa324.
Henderson NC, Rieder F, Wynn TA. Fibrosis: from mechanisms to medicines. Nature. 2020 Nov;587(7835):555-566. doi: 10.1038/s41586-020-2938-9.
Jiang W, Xiong Y, Li X, Yang Y. Cardiac Fibrosis: Cellular Effectors, Molecular Pathways, and Exosomal Roles. Front Cardiovasc Med. 2021 Aug 16;8:715258. doi: 10.3389/fcvm.2021.715258.
Landry, N.M., Rattan, S.G. & Dixon, LM.C. An Improved Method of Maintaining Primary Murine Cardiac Fibroblasts in Two-Dimensional Cell Culture. Sci Rep 9, 12889 (2019). https://doi.org/10.1038/s41598-019-49285-9.
Liang, Ch-Ch., Park, A.Y., Guan, J-L. In vitro scratch assay: a convenient and inexpensive method for analysis of cell migration in vitro. Nature Protocols 2007, 2:329-333.
Wei X, Zhang H. Four and a half LIM domains protein 1 can be as a double-edged sword in cancer progression. Cancer Biol Med. 2020 May 15;17(2):270-281. doi: 10.20892/j.issn.2095-3941.2019.0420. PMID: 32587768; PMCID: PMC7309467.

## Claims

1. A method for generation of immortalized cardiac fibroblast *Fhl1* knockout cells, comprising the steps:
a) obtaining primary cardiac fibroblasts,
b) Immortalizing the isolated fibroblasts, and
c) Producing *Fhl1* gene knockout in the cells.

2. The method according to claim 1, wherein the cardiac fibroblasts are mammal cardiac fibroblasts.

3. The method according to claim 2, wherein the cardiac fibroblasts are rodent cardiac fibroblasts.

4. The method according to claim 3, wherein the cardiac fibroblasts are mouse cardiac fibroblasts.

5. The method according to any one of claims 1 to 4, wherein after step a) the cells are cultured on a surface coated with PDMS and vitronectin.

6. The method according to any one of claims 1 to 5, wherein the cardiac fibroblasts are immortalized through viral-mediated induction of the large T-antigen.

7. The method according to claim 6, wherein the viral-mediated induction of the large T-antigen is made by using SV40 large T antigen.

8. The method according to any one of claims 1 to 7, wherein the *Fhl1* gene knockout is produced using CRISPR-Cas system.

9. The method according to claim 8, wherein the CRISPR-Cas system is CRISPR-Cas9 nickase system.

10. An immortalized cardiac fibroblast *Fhl1* knockout cell obtained by the method according to any one of the claims from 1 to 9.
